# FASCICULE DE BREVET EUROPEEN

(11) **EP 0 468 840 B1**
(45) Date de publication et mention de la délivrance du brevet: **09.04.1997**
(21) Numéro de dépôt: 91401849.4
(22) Date de dépôt: 04.07.1991
(51) Int. Cl.: C07D 301/12, C07D 301/14, C07D 303/16, C07C 69/54, C07D 317/24, C07C 67/29, C07C 69/00

(54) **Procédé d'époxydation sélective de (méth)acrylates insaturés, nouveaux (méth)acrylates fonctionels obtenus et leur application à la synthèse de nouveaux polymères.**
Verfahren zur selektiven Epoxidierung von ungesättigten (Meth)Acrylaten, erhaltene neue funktionelle (Meth)Acrylate und ihre Verwendung in der Synthese von neuen Polymeren
Process for the selective epoxidation of unsaturated (meth)acrylates, new functional (meth)acrylates so obtained and their use in the synthesis of new polymers

(30) Priorité: 06.07.1990 FR 9008607
(43) Date de publication de la demande: 29.01.1992
(60) Demande divisionnaire: 96102424.7
(73) Titulaire: ELF ATOCHEM S.A., 92800 Puteaux (FR)
(72) Inventeur: Caubere, Paul, F-54000 Nancy (FR); Fort, Yves, F.54500 Vandoeuvre Les Nancy (FR); Ortar, Agnès, F-54800 Jarny (FR)
(74) Mandataire: Chaillot, Geneviève

(56) Documents cités:
- FR-A- 1 329 386
- US-A- 3 575 925

## Description

La présente invention se rapporte à l'époxydation sélective de (méth)acrylates insaturés, permettant d'accéder à des (méth)acrylates fonctionnels applicables à la synthèse de nouveaux polymères.

L'époxydation de composés organiques insaturés au moyen de peroxyde d'hydrogène et d'un sel d'acide de métal lourd transformé in situ en un sel de peracide correspondant est bien connue depuis longtemps. Ainsi, les brevets US-A-2 833 787 et 2 833 788 ont décrit l'époxydation de composés éthyléniques non conjugués, par exemple d'alcools monoéthyléniques, au moyen de peroxyde d'hydrogène et de pertungstate de sodium à un pH compris entre 3 et 7. De même, l'article publié dans J. Org. Chem., vol.24, pages 54-55 (janvier 1959) décrit l'époxydation d'acides α,β-insaturés au moyen de peroxyde d'hydrogène et de tungstate de sodium à un pH compris entre 4 et 5,5.

Il est également connu par Angew. Chem. Int. Ed. Engl. 21 (1982) 734-750 l'époxydation d'oléfines au moyen de peroxyde d'hydrogène et de composés de molybdène. De même, J. Org. Chem. (1983), vol. 48, 3831-3833 décrit l'époxydation d'oléfines à 70°C dans des conditions de catalyse par transfert de phase au moyen de peroxyde d'hydrogène dilué (à moins de 10%) et de tungstate alcalin soluble dans l'eau, à pH 1,6 et en présence d'un agent de transfert de phase tel qu'un halogénure d'ammonium ou de phosphonium quaternaire, le rapport molaire du peroxyde d'hydrogène à l'oléfine étant égal à 0,6. L'article publié par J. Org. Chem. (1985) vol. 50, 2688-2690 décrit une réaction similaire effectuée à 50°C en présence de complexes de molybdène et de ligands monodentates, le rapport molaire de peroxyde d'hydrogène à l'oléfine étant égal à 0,2.

Enfin, il est connu d'époxyder les oléfines à 60°C dans le chloroforme, en présence d'un catalyseur formé à partir d'acide molybdophosphorique ou tungstophosphorique et de chlorure de cétylpyridinium, au moyen de peroxyde d'hydrogène concentré (35%) et le rapport molaire du peroxyde d'hydrogène à l'oléfine étant égal à 1,5 ou à 1 : on pourra lire à ce sujet J. Org. Chem. (1987), vol. 52, 1868-1870 et J. Org. Chem. (1988), vol. 53, 3587-3593.

Par ailleurs, le brevet US-A-3 459 775 décrit l'époxydation de (méth)acrylate de vinylnorbornène à la température de 50°C, pendant une durée de 7 à 9 heures, au moyen d'acide peracétique. Le 2-époxyéthylbicyclo[2.2.1] hept-5(6)-yl (méth)acrylate est obtenu dans ces conditions avec un rendement ne dépassant pas 42%. De même, la demande de brevet JP-A-62/81 378 décrit l'époxydation de l'acrylate de dicyclopentényloxyéthyle à la température de 60°C, pendant 2 heures, au moyen de peroxyde d'hydrogène à une concentration de 35%. L'acrylate époxydé est obtenu dans ces conditions avec un rendement ne dépassant pas 48%.

GB-A-2 055 821 décrit un procédé pour l'époxydation catalytique d'oléfines par réaction avec le peroxyde d'hydrogène selon la technique à double phase avec des sels d'onium, la réaction étant conduite dans un système liquide à deux phases aqueuse-organique, qui comprend une phase organique contenant essentiellement l'oléfine et une phase acide aqueuse contenant essentiellement le peroxyde d'hydrogène, en présence d'un système catalytique comprenant un premier composant catalytique qui est au moins l'un parmi W, Mo et V et leurs dérivés, et un second composant catalytique qui est un dérivé du phosphore ou de l'arsenic.

Le problème que la présente invention s'est proposé de résoudre consiste, compte tenu des enseignements de l'art antérieur rappelé ci-dessus relatifs à l'époxydation de composés organiques à insaturation éthylénique tels que des oléfines, des alcools, des acides ou certains (méth)acrylates, à mettre au point les conditions de l'époxydation sélective d'insaturations non acryliques de composés (méth)acryliques insaturés, en particulier au moyen de peroxyde d'hydrogène. Au cours des travaux ayant conduit à la présente invention a également été réalisée avec succès l'époxydation de certains (méth)acrylates insaturés au moyen de peracides organiques. Toutefois cette dernière méthode s'est révélée d'une portée moins générale que celle ayant recours au peroxyde d'hydrogène et elle oblige d'autre part à éliminer en fin d'opération l'acide organique formé par la réaction.

Ainsi un premier objet de la présente invention consiste en un procédé d'époxydation d'un composé (méth)acrylique insaturé de formule générale : 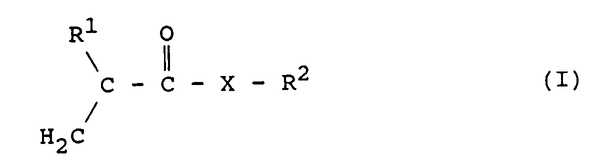 dans laquelle :
- X est choisi parmi les atomes d'oxygène et de soufre, le radical NH, les radicaux NR³ dans lesquels R³ est un groupe alkyle ayant de 1 à 12 atomes de carbone, et les radicaux oxyalkylènes O-(CH₂)ₙ dans lesquels n est un nombre entier allant de 1 à 16 environ,
- R² désigne une chaîne hydrocarbonée polycyclique comprenant jusqu'à 20 atomes de carbone et ayant une insaturation éthylénique exo- ou endocyclique, ou un hétérocycle mono- ou polycyclique comprenant jusqu'à 20 atomes de carbone et ayant une insaturation éthylénique exo- ou endocyclique, R² pouvant également désigner un reste dicyclopentényloxyéthyle, et
- R¹ est choisi parmi l'atome d'hydrogène et les radicaux alkyles ayant de 1 à 5 atomes de carbone,
ladite époxydation s'effectuant majoritairement sur l'insaturation éthylénique non (méth)acrylique,
par action sur ledit (méth)acrylate insaturé, à une température comprise entre 10°C et 60°C environ, du peroxyde d'hydrogène en présence d'au moins un hétéropolyacide.

Un très grand nombre de composés (méth)acryliques insaturés peuvent être époxydés conformément au procédé selon l'invention. On peut citer notamment les acrylates et méthacrylates de dicyclopentényloxyéthyle, de vinylnorbornyle, de dicyclopentényle, d'éthylidènenorbornyle, etc.

De préférence le (méth)acrylate insaturé de formule (I) est stabilisé, avant réaction, par l'addition d'une quantité efficace d'au moins un composé tel que l'hydroquinone, le monoéther méthylique de l'hydroquinone, la phénothiazine, la N,N-diéthylhydroxylamine, le nitrobenzène, le di-tertiobutylcatéchol, le p-anilinophénol, le phosphite de di-(2-éthylhexyl)-octylphényle, le bleu de méthylène et leurs mélanges en toutes proportions. Par quantité efficace on entend une proportion généralement comprise entre 0,05 et 0,5% environ en poids du méthacrylate insaturé de formule (I).

La réaction à la base du procédé selon l'invention doit être effectuée à une température modérée en évitant les températures supérieures à 50°C environ, qui sont souvent la cause de la polymérisation du composé (méth)acrylique insaturé ou de son époxyde, et les températures inférieures à 10°C environ, pour des raisons cinétiques. La réaction selon l'invention est effectuée de préférence en présence d'un solvant, ou d'un mélange de solvants, organique qui peut être choisi notamment parmi les solvants chlorés, tels que le dichloro-1,2-éthane, le chloroforme, le tétrachlorure de carbone, le trichlorométhane ou le dichlorométhane, et les solvants aromatiques tels que le benzène et le toluène, et en quantité telle que le rapport en volume composé à époxyder/solvant soit supérieur à 1,5 environ.

Dans le procédé selon l'invention, le rapport molaire entre le peroxyde d'hydrogène et le composé (méth)acrylique insaturé a une grande importance : il est d'au moins une mole, de préférence 1,5 à 3 moles, de peroxyde d'hydrogène, pour une mole de composé (méth)acrylique insaturé. De même, la concentration du peroxyde d'hydrogène, utilisé en solution aqueuse, n'est pas sans influence sur le rendement et sur la sélectivité de la réaction d'époxydation: elle est de préférence comprise entre 5% et 50% environ, et plus particulièrement comprise entre 10% et 35%. Une autre condition réactionnelle importante est le pH du milieu : il sera de préférence choisi entre 1,0 et 3,5 environ, et plus particulièrement entre 1,5 et 2,5, et sera ajusté au moyen de la quantité nécessaire d'un acide tel que l'acide phosphorique et/ou l'acide sulfurique.

L'hétéropolyacide utilisé selon l'invention en association avec le peroxyde d'hydrogène est notamment un hétéropolyacide ayant pour formule générale :

HₙAₐD_{c}O_{y}.xH₂O

dans laquelle :
- A représente le phosphore, le bore, le silicium, le germanium, l'étain, l'arsenic, l'antimoine, le cuivre, le nickel, le cobalt, le fer, le cérium, le thorium, le chrome ou un mélange d'au moins deux de ces éléments ;
- D représente le molybdène, le tungstène, le vanadium ou une combinaison d'au moins deux de ces éléments ;
- a est un nombre de 0,1 à 10 ;
- c est un nombre de 6 à 18 ;
- n est le nombre d'hydrogènes acides dans l'hétéropolyacide et est un nombre supérieur à 1 ;
- y est le nombre d'oxygènes dans l'hétéropolyacide et est un nombre de l'ordre de 10 à 70 ; et
- x est le nombre de moles d'eau de cristallisation et est un nombre de O à environ 40.

Ces hétéropolyacides sont connus et ils peuvent être préparés par des techniques connues. Des hétéropolyacides particulièrement intéressants sont l'acide phosphomolybdique (H₃PMo₁₂O₄₀), l'acide phosphotungstique (H₃PW₁₂O₄₀) et l'acide silicotungstique (H₄SiW₁₂O₄₀).

La réaction selon l'invention conduit, avec un taux de conversion le plus souvent supérieur à 70%, de manière sélective à l'époxydation de la double liaison oléfinique de la chaîne R² de manière à former un époxyde (méth)acrylique que l'on pourrait représenter par la formule : 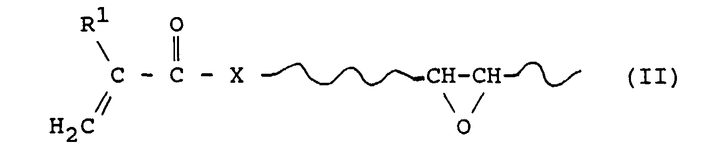 dans laquelle R¹ et X ont la signification précitée. Celui-ci est formé préférentiellement à l'époxyde de formule : 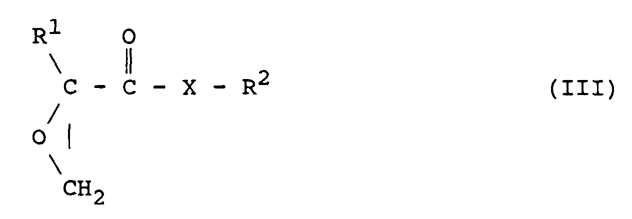 résultant de l'époxydation de la double liaison acrylique, qui peut néanmoins se former de manière notable dans des conditions particulières, ainsi que de manière plus rare le diépoxyde de formule : 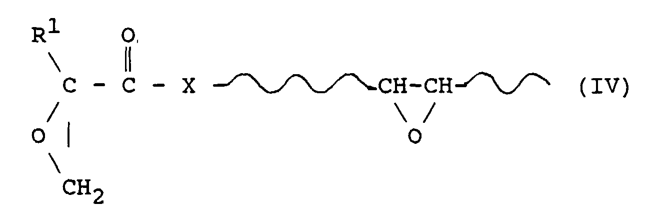 résultant de l'époxydation simultanée des deux doubles liaisons oléfinique et acrylique. Ce résultat de l'invention est particulièrement surprenant dans la mesure où jusqu'ici, conformément aux documents antérieurs déjà cités, l'époxydation de (méth)acrylates insaturés procédait avec de faibles rendements malgré des températures réactionnelles plus élevées que celles préconisées par la présente invention.

Parmi les (méth)acrylates époxydés que l'on peut obtenir selon l'invention, on peut citer notamment les (méth)acrylates époxydés de formules : 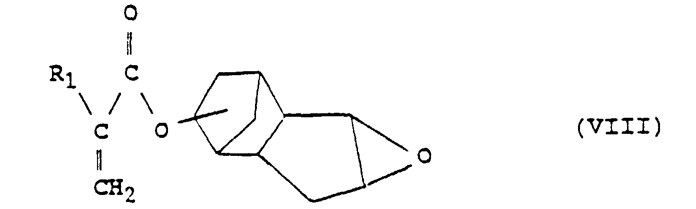 et 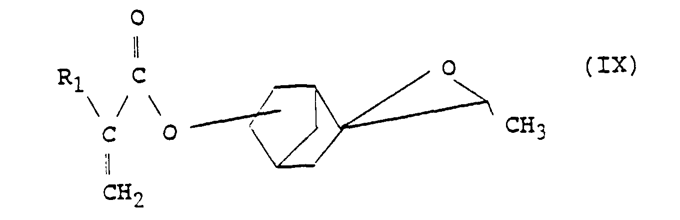 dans lesquelles R₁ est choisi parmi l'atome d'hydrogène et le radical méthyle.

Ces (méth)acrylates époxydés de formules (VIII) et (IX) sont respectivement les acrylate et méthacrylate d'octahydro-2,5-méthano-2H-undéno[1,2-b]oxyrényle et d'hydroxy-5(6)[époxy-2,1' éthyle]-2 bicyclo[2.2.1]heptane.

Les (méth)acrylates époxydés selon l'invention sont des composés possédant une faible odeur. Ils se polymérisent facilement et se copolymérisent aussi avec des monomères à insaturation éthylénique, tels que l'éthylène, ainsi que :
- un acrylate ou méthacrylate d'alkyle dont le groupe alkyle linéaire ou ramifié, le cas échéant substitué, par exemple par au moins un atome d'halogène comme le chlore ou le fluor et/ou par au moins un groupe hydroxyle, possède de 1 à 20 atomes de carbone,
- un acrylate ou méthacrylate d'aryle tel que le méthacrylate de benzyle,
- un hydrocarbure vinylaromatique tel que le styrène, le vinyltoluène, l'alphaméthylstyrène, le méthyl-4 styrène, le méthyl-3 styrène, le méthoxy-4 styrène, l'hydroxyméthyl-2 styrène, l'éthyl-4 styrène, l'éthoxy-4 styrène, le diméthyl-3,4 styrène, le chloro-2 styrène, le chloro-3 styrène, le chloro-4 méthyl-3 styrène, le tert.-butyl-3 styrène, le dichloro-2,4 styrène, le dichloro-2,6 styrène et le vinyl-1 naphtalène,
- un nitrile insaturé tel que l'acrylonitrile ou le méthacrylonitrile,
- une maléimide N-substituée telle que la N-éthylmaléimide, la N-isopropylmaléimide, la N-n-butylmaléimide, la N-isobutylmaléimide, la N-terbutylmaléimide, la N-n-octylmaléimide, la N-cyclohexylmaléimide, la N-benzylmaléimide et la N-phénylmaléimide,
- un anhydride d'acide dicarboxylique insaturé tel que l'anhydride maléique, l'anhydride itaconique, l'anhydride citraconique ou l'anhydride tétrahydrophtalique,
- l'acide acrylique ou méthacrylique,
- un acrylate ou méthacrylate de polyol comme les diacrylates et diméthacrylates de l'éthylèneglycol, du propylèneglycol, du 1,3-butanediol, du 1,4-butanediol, du 1,6-hexanediol, du néopentylglycol, du 1,4-cyclohexanediol, du 1,4-cyclohexanediméthanol, du 2,2,4-triméthyl-1,3-pentanediol, du 2-éthyl-2-méthyl-1,3-propanediol, du 2,2-diéthyl-1,3-propanediol, du diéthylèneglycol, du dipropylèneglycol, du triéthylèneglycol, du tripropylèneglycol, du tétraéthylèneglycol, du tétrapropylèneglycol, du triméthyloléthane, du triméthylolpropane, du glycérol, du pentaérythritol, les triacrylates et triméthacrylates du triméthyloléthane, du triméthylolpropane, du glycérol, du pentaérythritol, les tétraacrylates et tétraméthacrylates du pentaérythritol, les di(méth)acrylates à hexa(méth)acrylates du dipentaérythritol, les poly(méth)acrylates de polyols mono- ou polyéthoxylés ou mono- ou polypropoxylés tels le triacrylate et le triméthacrylate du triméthylolpropane triéthoxylé, du triméthylolpropane tripropoxylé ; le triacrylate et le triméthacrylate du glycérol tripropoxylé ; le triacrylate, le triméthacrylate, le tétraacrylate et le tétraméthacrylate du pentaérythritol tétraéthoxylé,
- un acrylamide ou méthacrylamide, un acrylate ou méthacrylate de dialkylaminoalkyle et leurs sels quaternaires,
   - l'acrylate et le méthacrylate de (norbornyloxy-2)-2 éthyle et de (diméthanodécahydronaphtyloxy-2)-2 éthyle, et
- des oxazolidones acryliques et méthacryliques choisies parmi celles de formule : 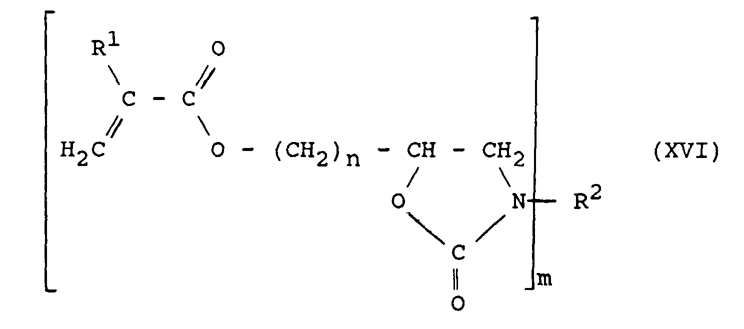 et celles de formule : 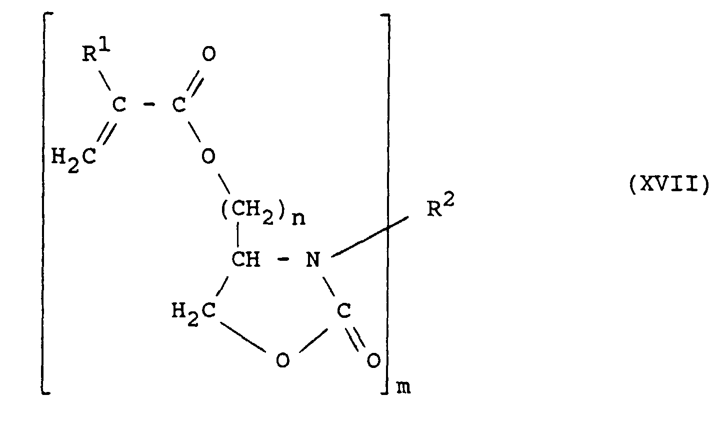 formules dans lesquelles :
- R¹ est choisi parmi l'atome d'hydrogène et le radical méthyle,
- n est un nombre entier allant de 1 à 12,
- m est un nombre entier allant de 1 à 3, et
- R² est un radical hydrocarboné, alkyle linéaire, ramifié ou cyclique ou bien aromatique, possédant de 5 à 12 atomes de carbone,
   lesdites oxazolidones pouvant être obtenues par réaction, entre 30°C et 90°C, d'un composé portant une fonction (méth)acrylique avec un composé portant au moins une fonction isocyanate,
- des composés acryliques et méthacryliques choisis parmi ceux de formule : 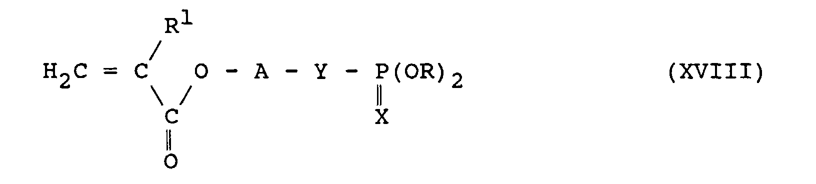 dans laquelle :
- R¹ est choisi parmi l'atome d'hydrogène et le radical méthyle,
- A est choisi parmi les radicaux (CH₂)ₙ pour lesquels n est un nombre entier de 2 à 12 et le radical -(CH₂CH₂O)_{d}-CH₂CH₂-, d étant un nombre entier allant de 1 à 20,
- X est choisi parmi les atomes de soufre et d'oxygène,
- Y est choisi parmi les atomes de soufre et d'oxygène, avec la condition que X est un atome de soufre et Y est un atome d'oxygène lorsque A est le radical -(CH₂CH₂O)_{d}- CH₂CH₂-, et
- R est choisi parmi les radicaux alkyle ayant de 1 à 20 atomes de carbone et les groupes -(CH₂)ₚSR³ dans lesquels p est un nombre entier allant de 3 à 12 et R³ est un radical alkyle ayant de 1 à 20 atomes de carbone,
   ceux de formule : 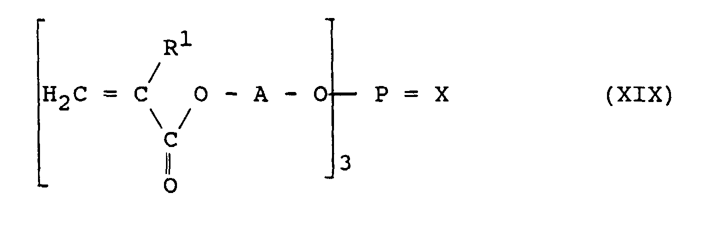 dans laquelle :
- R¹ est choisi parmi l'atome d'hydrogène et le radical méthyle,
- A est choisi parmi les radicaux (CH₂)ₙ pour lesquels n est un nombre entier de 2 à 12 et le radical -(CH₂CH₂O)_{d}-CH₂CH₂-, d étant un nombre entier allant de 1 à 20, et
- X est choisi parmi les atomes de soufre et d'oxygène, et ceux de formule : 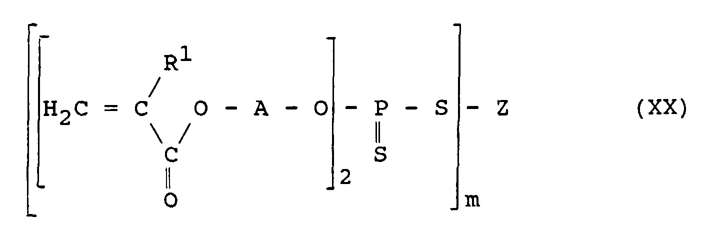 dans laquelle :
- R¹ est choisi parmi l'atome d'hydrogène et le radical méthyle,
- A est choisi parmi les radicaux (CH₂)ₙ pour lesquels n est un nombre entier de 2 à 12,
- m est un nombre entier allant de 1 à 3, et
- Z est choisi parmi l'atome d'hydrogène, les radicaux R²QH, R² étant un radical alkyle ayant de 2 à 12 atomes de carbone et Q étant choisi parmi les atomes d'oxygène et de soufre, et les atomes des métaux des groupes IA, IIA, IIIA, IB, IIB, VIB, VIIB et VIII de la Classification Périodique,
   avec la condition que Z est choisi parmi l'atome d'hydrogène et les radicaux R²OH lorsque m = 1 et que m est la valence de Z lorsque Z est un métal.
   De tels composés peuvent être préparés par réaction d'un composé acrylique ou méthacrylique de formule : 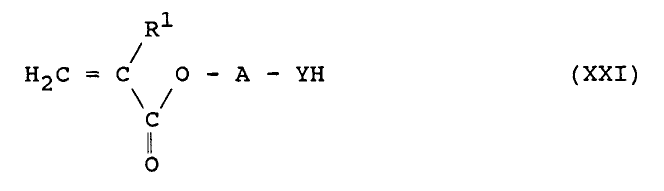 dans laquelle R¹, A et Y ont les mêmes significations que dans la formule (X), avec un composé pentavalent du phosphore, celui-ci pouvant être par exemple un composé de formule PXT₃ dans laquelle X a la même signification que dans la formule (X) et T désigne un atome d'halogène, ou bien un composé phosphoré de formule : 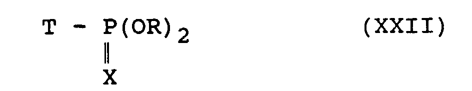 dans laquelle R et X ont les mêmes significations que dans la formule (I) et T désigne un atome d'halogène, ou bien le pentasulfure P₂S₅,
- des composés acryliques et méthacryliques choisis parmi ceux de formule : 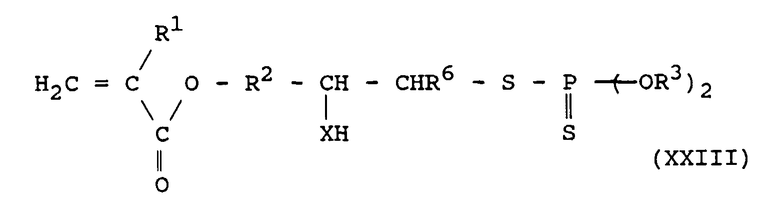 et ceux de formule : 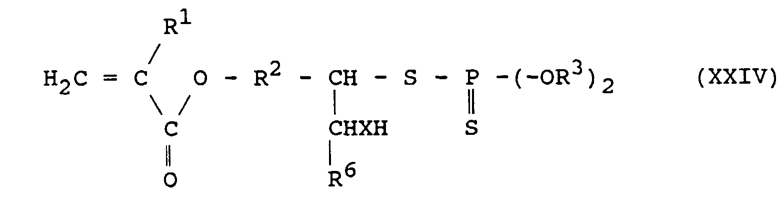 formules dans lesquelles :
- R¹ est choisi parmi l'atome d'hydrogène et le radical méthyle,
- X est un hétéroatome choisi parmi l'oxygène et le soufre,
- R² est choisi parmi les groupes alkylènes linéaires ou ramifiés, cycloalkylènes et hétérocycloalkylènes mono-ou polycycliques, alkylarylènes et arylalkylènes comprenant de 1 à 12 atomes de carbone,
- R⁶ est choisi parmi l'atome d'hydrogène et les radicaux alkyles et aryles ayant de 1 à 12 atomes de carbone, et
- R³ est choisi parmi les radicaux alkyles et aryles ayant de 1 à 20 atomes de carbone, les groupes -(CH₂)ₚSR⁴ dans lesquels p est un nombre entier allant de 2 à 12 et R⁴ est un radical alkyle ayant de 1 à 20 atomes de carbone ou bien un groupe cycloalkyle mono- ou poly-cyclique ayant de 4 à 10 atomes de carbone, chaque cycle dudit groupe comprenant de 4 à 6 chaînons, et les groupes 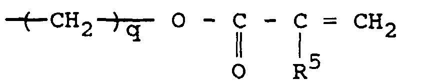 dans lesquels q est un nombre entier allant de 2 à 12 et R⁵ est choisi parmi l'atome d'hydrogène et le radical méthyle. De tels composés peuvent être préparés par réaction d'un époxyde ou épisulfure acrylique ou méthacrylique de formule : 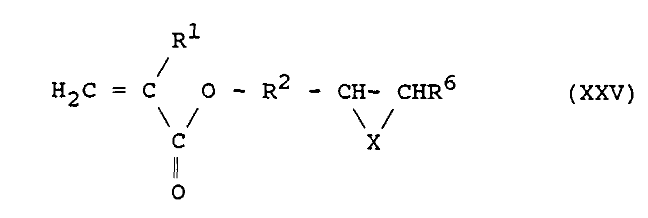 dans laquelle R¹, R², R⁶ et X ont les mêmes significations que dans la formule (XI),
   avec un composé thiophosphoré de formule :

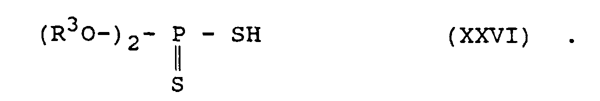

De tels polymères et copolymères sont obtenus en (co)polymérisant au moins un composé acrylique soufré selon l'invention et, le cas échéant, au moins un comonomère copolymérisable, tel que défini précédemment, en présence d'au moins un initiateur de radicaux libres tel qu'un peroxyde, un hydroperoxyde ou un composé diazo. La (co)polymérisation est généralement effectuée à une température comprise entre 50°C et 120°C environ et en utilisant l'un des monomères comme solvant. Elle peut également s'effectuer en émulsion dans l'eau, à une température comprise entre 50°C environ et 100°C, en présence d'au moins un agent tensio-actif.

Lorsque le comonomère est l'éthylène, on peut utiliser une température de 140° à 300°C environ et une pression de 1000 à 3000 bars environ. Lorsque le comonomère est un hydrocarbure vinylaromatique, on peut utiliser une température de 80° à 200°C environ.

Les époxydes (méth)acryliques selon l'invention, en raison de leur faible odeur et de leur basse viscosité, pourront trouver des applications en tant qu'agents de modification dans les domaines des encres, des adhésifs, des peintures, des revêtements et des résines.

Les exemples suivants sont donnés à titre illustratif et non limitatif de la présente invention.

### EXEMPLE 1

Un mélange de 0,24 millimole d'acide tungstophosphorique H₃PW₁₂O₄₀ et de 120 millimoles de peroxyde d'hydrogène 35% aqueux est additionné goutte à goutte, en 15 minutes, à une solution de 60 millimoles de méthacrylate de dicyclopentényloxyéthyle dans 15 ml de chloroforme. Le mélange est placé sous vive agitation à 45°C. Après 3 heures la réaction est terminée en ajoutant une solution acide de sulfate de fer au mélange réactionnel afin de détruire les peroxydes présents dans le milieu. A ce stade sont calculés le taux de conversion de la réaction C (exprimé en %) ainsi que les sélectivités S en époxyde de formule (II) et en époxyde de formule (III). Les résultats sont les suivants : C = 100 ; S(II) = 100 ; et S(III) = 0.

### EXEMPLES 2 à 4

Dans un réacteur on introduit x grammes de (méth)acrylate époxydé et y grammes de méthacrylate de méthyle puis 0,2 gramme d'azobisisobutyronitrile. Après l'avoir purgé à l'azote, on plonge le réacteur dans un bain à 83°C. On enregistre la température (exprimée en degrés Celsius) Tₑₓₒ du pic exothermique puis on effectue, après retour à la température du bain thermostatique (83°C) une cuisson isotherme pendant 5 heures. En sortie de réacteur, le polymère obtenu est lavé au méthanol, broyé puis séché sous vide de 10 mmHg à 40°C. Le tableau I ci-après indique, outre le rendement (Rdt) en polymère exprimé en pourcentage, la température de transition vitreuse Tg du polymère (exprimée en degrés Celsius) ainsi que la solubilité du polymère dans différents solvants, exprimée soit en pourcentage pondéral soit par une observation visuelle (gonflement par exemple).

L'époxyde utilisé dans les exemples suivants est soit le méthacrylate de dicyclopentényloxyéthyle (Exemple 2) soit l'acrylate de dicyclopentényloxyéthyle (Exemples 3 et 4).

**Tableau I**

| Exemple | 2 | 3 | 4 | 5 |
|---|---|---|---|---|
| x | 10 | 10 | 6 | 5 |
| y | 0 | 0 | 4 | 5 |
| Tₑₓₒ | 133 | 118 | nd | - |
| Rdt | 90 | 80 | 85 | 85 |
| Tg | +45 | +39 | +60 | +75 |
| acétone | gonfle | 0 | 50 | 50 |
| méthanol | gonfle | nd | nd | nd |
| heptane | 0 | 0 | 0 | 0 |
| acétate d'éthyle | 60 | 50 | 50 | 50 |
| nd : non déterminé | | | | |

### EXEMPLE 5

Dans un réacteur on introduit 5 grammes de méthacrylate de méthyle, 5 grammes de méthacrylate de dicyclopentényloxyéthyle, 10 grammes de méthyléthylcétone et 0,2 gramme d'azobisisobutyronitrile. Après l'avoir purgé par un courant d'azote, on le plonge dans un bain thermostatique à 83°C et on effectue une cuisson exotherme pendant 5 heures. En fin de réaction, le polymère est traité et caractérisé comme aux exemples 2 à 4 (voir tableau I).

### EXEMPLE 6

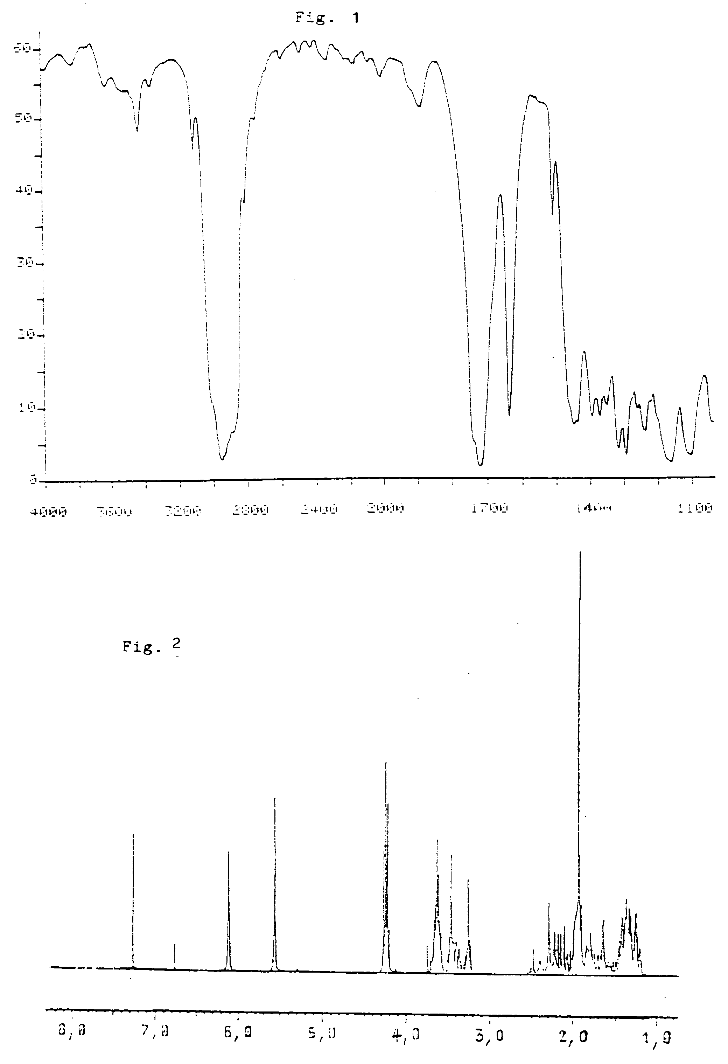

L'époxyde du méthacrylate de dicyclopentényloxyéthyle préparé à l'Exemple 1 a été caractérisé par :
- spectrophotométrie infrarouge au moyen d'un spectromètre PERKIN ELMER 841 : le spectre obtenu (figure 1) comporte des bandes caractéristiques à 2954 cm⁻¹, 1722 cm⁻¹, 1639 cm⁻¹, 1453 cm⁻¹, 1169 cm⁻¹, 1107 cm⁻¹, 941 cm⁻¹ et 836 cm⁻¹.
- résonance magnétique nucléaire du carbone 13 : déplacements chimiques à 167,1 ppm, 136,2 ppm, 125,7 ppm, 77,2-81,8 ppm, 65,9 ppm, 59,6-62,0 ppm, 26,0-52,0 ppm et 18,3 ppm.
- résonance magnétique nucléaire du proton au moyen d'un spectromètre JEOL PMX 60SI : le spectre obtenu (figure 2) montre des déplacements chimiques à 6,1 ppm (m, 1H), 5,6 ppm (m, 1H), 4,25 ppm (m, 2H), 3,2-3,7 ppm (m, 5H), et 1,2-2,5 ppm (m, 13H).

## Revendications

1. Procédé d'époxydation d'un composé (méth)acrylique insaturé de formule générale : 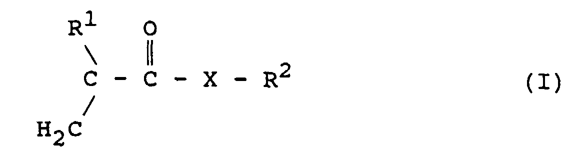 dans laquelle :
- R¹ est choisi parmi l'atome d'hydrogène et les radicaux alkyle ayant de 1 à 5 atomes de carbone ;
- X est choisi parmi les atomes d'oxygène et de soufre, le radical NH, les radicaux NR³ dans lesquels R³ est un groupe alkyle ayant de 1 à 12 atomes de carbone, et les radicaux O-(CH₂)ₙ dans lesquels n est un nombre entier allant de 1 à 16 environ ; et
- R² désigne une chaîne hydrocarbonée polycyclique ayant jusqu'à 20 atomes de carbone et ayant une insaturation éthylénique endo- ou exocyclique, ou un hétérocycle mono- ou polycyclique comprenant jusqu'à 20 atomes de carbone et ayant une insaturation éthylénique exo- ou endocyclique, R² pouvant également désigner un reste dicyclopentényloxyéthyle,
ladite époxydation s'effectuant majoritairement sur l'insaturation éthylénique non (méth)acrylique, par action sur ledit (méth)acrylate insaturé, à une température comprise entre 10°C et 60°C environ, du peroxyde d'hydrogène en présence d'au moins un hétéropolyacide.

2. Procédé selon la revendication 1, caractérisé en ce que la réaction est effectuée en présence d'un solvant chloré ou aromatique.

3. Procédé d'époxydation selon l'une des revendications 1 et 2, caractérisé en ce que ledit hétéropolyacide a pour formule générale :
HₙAₐD_{c}O_{y}.xH₂O
dans laquelle :
- A représente le phosphore, le bore, le silicium, le germanium, l'étain, l'arsenic, l'antimoine, le cuivre, le nickel, le cobalt, le fer, le cérium, le thorium, le chrome ou un mélange d'au moins deux de ces éléments ;
- D représente le molybdène, le tungstène, le vanadium ou une combinaison d'au moins deux de ces éléments ;
- a est un nombre de 0,1 à 10 ;
- c est un nombre de 6 à 18 ;
- n est le nombre d'hydrogènes acides dans l'hétéropolyacide et est un nombre supérieur à 1 ;
- y est le nombre d'oxygènes dans l'hétéropolyacide et est un nombre de l'ordre de 10 à 70 ; et
- x est le nombre de moles d'eau de cristallisation et est un nombre de 0 à environ 40.

4. Procédé d'époxydation selon l'une des revendications 1 à 3, caractérisé par le fait que le composé (méth)acrylique de formule (I) est le (méth)acrylate de dicyclopentényloxyéthyle.

5. Procédé selon l'une des revendications 1 à 4, caractérisé par le fait que l'hétéropolyacide est l'acide tungstophosphorique H₃PW₁₂O₄₀.

## Claims

1. Process for the epoxidation of an unsaturated (meth)acrylic compound of general formula : 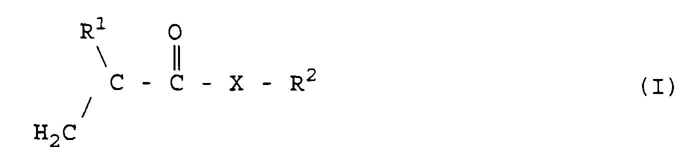 in which :
- R¹ is chosen from a hydrogen atom and alkyl radicals having from 1 to 5 carbon atoms ;
- X is chosen from oxygen and sulphur atoms, the NH radical, the radicals NR³ in which R³ is an alkyl group having from 1 to 12 carbon atoms, and the radicals O-(CH₂)ₙ in which n is an integer ranging from 1 to 16 approximately ; and
- R² denotes a polycyclic hydrocarbon chain comprising up to 20 carbon atoms and having an endo- or exocyclic ethylenic unsaturation, or a mono- or polycyclic heterocycle comprising up to 20 carbon atoms and having an endo- or exocyclic ethylenic unsaturation, wherein R² can also designate a dicyclopentenyloxyethyl rest ;
said epoxidation being mostly carried out on the non (meth)acrylic ethylenic unsaturation, by the action of hydrogen peroxide, on said unsaturated (meth)acrylate, at a temperature of between 10°C and 60°C approximately, in the presence of at least one heteropolyacid.

2. Process according to Claim 1, characterized in that the reaction is carried out in the presence of a chlorinated or aromatic solvent.

3. Epoxidation process according to any one of Claims 1 and 2, characterized in that said heteropolyacid has the general formula :
HₙAₐD_{c}O_{y}.xH₂O
in which :
- A represents phosphorus, boron, silicon, germanium, tin, arsenic, antimony, copper, nickel, cobalt, iron, cerium, thorium, chromium or a mixture of at least two of these elements ;
- D represents molybdenum, tungsten, vanadium or a combination of at least two of these elements ;
- a is a number from 0.1 to 10 ;
- c is a number from 6 to 18 ;
- n is the number of acid hydrogens in the heteropolyacid and is a number higher than 1 ;
- y is the number of oxygens in the heteropolyacid and is a number of the order of 10 to 70 ; and
- x is the number of moles of water of crystallisation and is a number of from 0 to about 40.

4. Epoxidation process according to any one of Claims 1 to 3, characterized in that the (meth)acrylic compound of formula (I) is dicyclopentenyloxyethyl (meth)acrylate.

5. Process according to any one of Claims 1 to 4, characterised in that the heteropolyacid is phosphotungstic acid H₃PW₁₂O₄₀,

## Patentansprüche

1. Verfahren zur Epoxidierung einer ungesättigten (Meth-)Acrylverbindung der allgemeinen Formel: 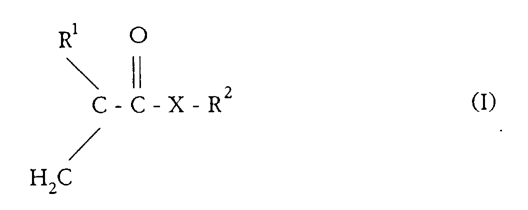 in der:
- R¹ ausgewählt ist aus einem Wasserstoffatom und Alkylresten mit 1 bis 5 Kohlenstoffatomen;
- X ausgewählt ist aus einem Sauerstoffatom, einem Schwefelatom, einem NH-Rest, NR³-Resten, in denen R³ eine Alkylgruppe mit 1 bis 12 Kohlenstoffatomen ist, und O-(CH₂)ₙ-Resten, in denen n eine ganze Zahl von 1 bis etwa 16 ist; und
- R² eine polycyclische Kohlenwasserstoffkette mit bis zu 20 Kohlenstoffatomen und mit einer endo- oder exocyclischen, ethylenisch ungesättigten Bindung oder einen mono- oder polycyclischen Heterocyclus bezeichnet, der bis zu 20 Kohlenstoffatome umfaßt und eine exo- oder endocyclische, ethylenisch ungesättigte Bindung enthält, wobei R² ebenfalls einen Dicyclopentenyloxyethylrest bezeichnen kann,
wobei die Epoxidierung hauptsächlich an der nicht(meth-)acrylischen, ethylenisch ungesättigten Bindung erfolgt, und zwar durch Einwirkung von Wasserstoffperoxid in Gegenwart mindestens einer Heteropolysäure auf das ungesättigte (Meth-)Acrylat bei einer Temperatur zwischen etwa 10 °C und 60 °C.

2. Verfahren nach Anspruch 1, dadurch gekennzeichnet, daß die Reaktion in Gegenwart eines chlorierten oder aromatischen Lösemittels durchgeführt wird.

3. Verfahren zur Epoxidierung nach Anspruch 1 oder 2, dadurch gekennzeichnet, daß die Heteropolysäure die allgemeine Formel:
HₙAₐD_{c}O_{y}·xH₂O
hat, in der:
- A Phosphor, Bor, Silicium, Germanium, Zinn, Arsen, Antimon, Kupfer, Nickel, Cobalt, Eisen, Cer, Thorium, Chrom oder eine Mischung von mindestens zwei dieser Elemente darstellt;
- D Molybdän, Wolfram, Vanadium oder eine Kombination von mindestens zwei dieser Elemente darstellt;
- a eine Zahl von 0,1 bis 10 ist;
- c eine Zahl von 6 bis 18 ist;
- n die Zahl der sauren Wasserstoffatome in der Heteropolysäure bezeichnet und eine Zahl größer als 1 ist;
- y die Zahl der Sauerstoffatome in der Heteropolysäure bezeichnet und eine Zahl in der Größenordnung von 10 bis 70 ist; und
- x die Zahl der Mol an Kristallwasser darstellt und in der Größenordnung von 0 bis etwa 40 liegt.

4. Verfahren zur Epoxidierung nach einem der Ansprüche 1 bis 3, dadurch gekennzeichnet, daß die (Meth-)Acrylverbindung der Formel (I) Dicyclopentenyloxyethyl(meth-)acrylat ist.

5. Verfahren nach einem der Ansprüche 1 bis 4, dadurch gekennzeichnet, daß die Heteropolysäure Phosphowolframsaüre H₃PW₁₂O₄₀ ist.
